Europäisches Patentamt

European Patent Office

Office européen des brevets

(19)

(11) Numéro de publication : **0 302 771 B1**

(12) # FASCICULE DE BREVET EUROPEEN

(45) Date de publication du fascicule du brevet :
**16.10.91 Bulletin 91/42**

(51) Int. Cl.⁵ : **C07C 11/00, C07C 7/148**

(21) Numéro de dépôt : **88401870.6**

(22) Date de dépôt : **20.07.88**

(54) **Procédé pour l'élimination conjointe d'arsenic et d'oxysulfure de carbone d'une coupe d'hydrocarbures insaturés en phase liquide.**

(30) Priorité : **07.08.87 FR 8711347**

(43) Date de publication de la demande :
**08.02.89 Bulletin 89/06**

(45) Mention de la délivrance du brevet :
**16.10.91 Bulletin 91/42**

(84) Etats contractants désignés :
**AT BE DE GB NL**

(56) Documents cités :
**GB-A- 2 162 194**
**US-A- 3 782 076**

(73) Titulaire : **INSTITUT FRANCAIS DU PETROLE**
**4, Avenue de Bois-Préau**
**F-92502 Rueil-Malmaison (FR)**

(72) Inventeur : **Boitiaux, Jean-Paul**
**4, avenue des Ursulines**
**F-78300 Poissy (FR)**
Inventeur : **Cosyns, Jean**
**50, route d'Herbeville**
**F-78580 Maule (FR)**

EP 0 302 771 B1

## Description

Les divers procédés de craquage de coupes lourdes de pétrole tels que le craquage catalytique, la viscoréduction et la cokéfaction produisent des coupes légères fortement contaminées par divers composés contenant du soufre, de l'azote et de l'oxygène. A côté de ces impuretés, on détecte très souvent de l'arsenic. Les composés sulfurés sont le plus souvent $H_2S$ et des mercaptans. Dans le cas du craquage catalytique, on observe aussi la présence de CO, $CO_2$ et COS. Les composés azotés, présents dans les coupes légères, sont principalement l'ammoniac ou des amines légères. L'arsenic est lui-même aussi présent sous forme de composé dont la formule générale est $AsR_3$, R pouvant être un radical hydrocarboné tel que $CH_3$ ou un atome d'hydrogène.

On entend, ici, par coupes légères, celles qui sont gazeuses dans les conditions normales de pression et de température, c'est à dire les coupes $C_2$, $C_3$, $C_4$. Ces coupes sont généralement traitées pour en éliminer les composés sulfurés. En particulier, les coupes $C_3$ et $C_4$ subissent usuellement un traitement de lavage aux amines, suivi d'un traitement de lavage à la soude. Ces divers lavages éliminent quasi totalement $H_2S$, en partie seulement les composés sulfurés organiques tels que les mercaptans, mais n'extraient COS que de façon très incomplète.

Les composés d'arsenic également ne sont pas extraits par ces lavages et restent présents dans les coupes traitées.

D'une manière générale, les coupes $C_3$ et $C_4$, qui contiennent une forte proportion d'oléfines, constituent une matière première de valeur pour la fabrication de carburants ou de produits chimiques tels que, par exemple, certains polymères. Ces transformations impliquent des traitements catalytiques divers dans lesquels les catalyseurs sont plus ou moins rapidement empoisonnés par les composés sulfurés ou arséniés. Ainsi, par exemple, la coupe $C_3$ provenant du craquage catalytique en lit fluidisé (FCC) contient encore, après les traitements de lavage cités plus haut, des teneurs en COS de l'ordre de 1 à 50 ppm poids et en arsenic de l'ordre de 0,1 à 5 ppm poids.

Il est difficile de déterminer avec certitude le type de composé d'arsenic présent dans cette coupe. Cependant, on observe généralement que l'arsenic, après séparation par distillation des diverses coupes légères, se retrouve principalement dans les coupes $C_2$ et $C_3$.

On pense donc qu'il s'agit d'arsines légères de type $AsH_3$ ou $AsH_2CH_3$.

Les coupes $C_3$ provenant du craquage catalytique (FCC) ont généralement la composition pondérale suivante :

| Propane | 15 | - | 40 % pds |
| Propylène | 60 | - | 85 % " |
| Propyne | 10 | - | 100 ppm |
| Propadiène | 10 | - | 100 " |
| $C_4^+$ | 0,1 | - | 2 % pds |
| COS | 1 | - | 50 ppm |
| As | 0,1 | - | 5 ppm |

Le propylène présent en forte proportion dans cette coupe incite les raffineurs à la valoriser le mieux possible, soit comme matière première pour la fabrication de carburant par dimérisation ou alkylation avec l'isobutane, soit comme produit de base pour la pétrochimie. Dans ce dernier cas, la coupe provenant du craquage catalytique est souvent valorisée en mélange avec des effluents de vapocraquage. Dans ces dernières coupes, la teneur en propyne et propadiène est beaucoup plus élevée et atteint généralement au total 4 à 7% (somme propyne + propadiène). Ces composés doivent être éliminés par hydrogénation sélective qui se fait généralement sur un catalyseur à base de palladium. Celui-ci est, alors, très rapidement empoisonné par l'arsenic et aussi le COS.

Il est connu, depuis longtemps, que l'oxyde de plomb est capable d'absorber l'arsenic. Plus récemment, on a décrit un procédé de captation de l'arsenic contenu dans les hydrocarbures gazeux utilisant une masse absorbante composée d'oxyde de plomb déposée sur une alumine de grande surface (brevet US 3.782.076). Il est recommandé, dans ce brevet, d'opérer à relativement basse pression (inférieure à 20 bars environ), c'est à dire, de fait, en phase gazeuse, de manière à éviter les réactions parasites de polymérisation qui seraient facilitées par une augmentation de pression. De plus, dans ce même brevet, il est recommandé d'éviter la pré-

2

sence d'H$_2$S qui apparaît comme diminuant l'efficacité d'absorption de la masse de captation.

On a aussi décrit l'emploi d'oxyde de plomb pour absorber l'oxysulfure de carbone, mais dans ce cas il faut exclure la présence d'arsenic. On opère ici aussi à basse pression, donc en phase gazeuse.

Contrairement à ces observations, il a, maintenant, été trouvé qu'il était possible d'absorber à la fois de l'arsenic et de l'oxysulfure de carbone à condition de travailler en phase liquide d'hydrocarbures, c'est à dire à des pressions relativement élevées (supérieures à 20 bars). En outre la désactivation du catalyseur, en phase liquide d'hydrocarbures est moins rapide qu'avec une phase gazeuse des réactifs, surtout quand la charge renferme des hydrocarbures acétyléniques et/ou dioléfiniques. Les résultats sont encore améliorés quand on utilise une masse absorbante renfermant de l'oxyde de plomb et un support "non acide", c'est à dire qu'il ne doit pas catalyseur les réactions connues par l'homme de l'art comme étant catalysées par les solides acides, à savoir les réactions d'isomérisation du squelette hydrocarboné, de craquage et de polymérisation. Ce support peut être, par exemple, de l'alumine, de la silice ou de la magnésie. On préfère, cependant, l'alumine avec laquelle on peut obtenir, à la fois, des surfaces spécifiques assez grandes et une résistance mécanique suffisante.

L'acidité du support peut être déterminée par le test connu d'adsorption d'ammoniac du type de celui décrit, par exemple, dans "Journal of Catalysis, 2, 211-222 (1963)" : la méthode consiste à chauffer le solide à 600°C sous vide (soit à une pression inférieure à environ 1 Pascal) jusqu'à dégazage total (ceci notamment pour enlever l'eau et les impuretés indésirables) ; ensuite, on place ce support dans un calorimètre à 320°C et on introduit une quantité d'ammoniac telle que la pression finale du système à l'équilibre atteigne environ 40 kPa et on mesure la quantité de chaleur dégagée. Mesurée, dans ces conditions, la chaleur d'adsorption d'ammoniac doit être inférieure à 40 joules par gramme de support.

Le support préconisé dans l'invention est de préférence une alumine d'une surface comprise entre 10 et 300 m$^2$/g et de préférence entre 50 et 200 m$^2$/g. Son volume poreux total est avantageusement compris entre 0,2 et 1,2 cm$^3$/g et de préférence entre 0,5 et 1,2 cm$^3$/g. Le volume macroporeux, défini comme étant celui correspondant aux pores supérieurs à 100 nm, est de préférence compris entre 0,1 et 0,5 cm$^3$/g.

Les supports d'alumine, communément utilisés pour divers hydrotraitements tels que l'hydrodésulfuration et l'hydrodéazotation, ou bien ceux utilisés pour la production d'essence par le procédé de reforming, ne conviennent pas dans la présente invention. En effet, ils présentent des chaleurs d'absorption d'ammoniac très nettement supérieures à 40 joules par gramme.

Les supports utilisés dans l'invention peuvent être obtenus de diverses manières. Une des manières préférées est celle qui consiste à autoclaver une alumine de transition d'une surface spécifique comprise entre 200 et 400 m$^2$/g sous pression de vapeur d'eau, puis à la calciner à haute température selon une méthode décrite dans les brevets FR 1.386.364, 1.449.904 et 1.584.460, de manière à obtenir une alumine d'une surface inférieure à 200 m$^2$/g et dont l'acidité mesurée par adsorption d'ammoniac est inférieure à 40 joules par gramme. Une autre manière préférée est celle qui consiste à imprégner un support d'alumine de transition (de surface spécifique comprise entre 200 et 400 m$^2$/g) avec un sel d'un métal du groupe VIII tel que le cobalt ou le nickel, et à calciner ensuite le solide imprégné à des températures de 700 à 850°C, selon une technique décrite dans le brevet FR 2.118.309, de manière à obtenir un produit ayant également une surface inférieure à 200 m$^2$/g et une acidité inférieure à 40 joules par gramme.

La masse absorbante contenant l'oxyde de plomb est préparée par mélange d'un composé de plomb avec le support, selon les techniques connues. La manière qui conduit à la masse la plus performante est l'imprégnation "à sec" : remplissage de la porosité du support avec un volume de liquide égal au volume poreux du support, le liquide étant, ici, une solution aqueuse d'un sel de plomb. Un sel de plomb aisément soluble est le nitrate de plomb ; on préfère, cependant, l'acétate de plomb qui possède une solubilité satisfaisante et permet d'obtenir une masse de captation d'efficacité substantiellement accrue.

Après imprégnation du support par la solution du composé de plomb, on chauffe jusqu'à une température comprise entre 300 et 700°C, et de préférence entre 400 et 550°C, pour convertir le composé de plomb en oxyde de plomb. On opère de préférence en atmosphère renfermant de l'oxygène.

Les masses obtenues renferment avantageusement de 5 à 50% en poids de plomb calculé en PbO.

L'absorption est conduite à une température comprise, de préférence, entre 10 et 100°C sous une pression suffisante pour maintenir la coupe à traiter en phase liquide. Le débit volumique horaire de charge liquide par volume de catalyseur est avantageusement compris entre 0,5 et 10 et de préférence entre 1 et 5. Les charges hydrocarbonées traitées dans l'invention, peuvent renfermer, par exemple 0,1 à 20% en poids d'hydrocarbures fortement, insaturés (acétyléniques et dioléfiniques), le plus souvent 1 à 10%, et 0,1 à 20 ppm d'arsenic, le plus souvent 0,5 à 5 ppm.

Les exemples suivants illustrent la présente invention.

Exemple 1.

Dans cet exemple, on a préparé différentes masses de captation par imprégnation à sec à partir d'une solution aqueuse d'acétate de plomb trihydraté et de différents supports d'alumine dont les caractéristiques principales sont données dans le tableau 1.

Tableau 1.

| Type de support | Surface spécifique $m^2/g$ | Volume poreux total $cm^3/g$ | Volume macroporeux $cm^3/g$ (1) | Acidité $\Delta$ H J/g (2) |
|---|---|---|---|---|
| Alumine gamma cubique | 205 | 0,60 | < 0,01 | 60 |
| Alumine autoclavée | 70 | 0,63 | 0,17 | 13,6 |
| Alumine autoclavée | 155 | 1,05 | 0,36 | 28 |
| alumine prétraitée (3) | 160 | 0,55 | 0,16 | 26 |

(1) Le volume macroporeux correspond au volume cumulé pour les pores de diamètre égal ou supérieur à 100 nm.

(2) L'acidité du support a été mesurée selon la méthode décrite précédemment dans ce brevet.

(3) L'alumine prétraitée a été préparée par imprégnation "à sec" d'une alumine de transition de 250 m²/g et d'un volume poreux total égal à 0,62 cm³/g avec une solution aqueuse de nitrate de nickel. La quantité de nickel introduite représentait 3% en poids par rapport au support. Le solide a été ensuite séché, puis calciné, sous courant d'air à pression atmosphérique à une température de 750°C pendant 2 heures.

La quantité d'acétate de plomb a été calculée pour obtenir des masses de captation renfermant 16% en poids d'oxyde de plomb calculé en PbO. Le solide imprégné est ensuite séché, puis traité sous courant d'air à 450°C pendant 2 heures. On a ainsi obtenu les quatre solides suivants :

| N° | Désignations |
|---|---|
| 1 | 16 % PbO/$Al_2O_3$ gamma cubique |
| 2 | 16 % PbO/$Al_2O_3$ autoclavée, 70 m²/g |
| 3 | 16 % PbO/$Al_2O_3$ autoclavée 155 m²/g |
| 4 | 16 % PbO/$Al_2O_3$ prétraitée |

On a réalisé, alors, une série de tests dans lesquels on a traité une coupe $C_3$ provenant en partie d'un vapocraquage et en partie d'une installation de craquage catalytique en lit fluide (FCC). La coupe à traiter avait

la composition suivante :

|  | % poids |
|---|---|
| Propane | 12,0 |
| Propylène | 83,2 |
| Propyne | 2,5 |
| Propadiène | 2,1 |
| $C_4$ | 0,2 |
| $C_5^+$ | non détectable |
| COS | 5 ppm |
| As | 2,9 ppm |

On a disposé 50 cm³ de la masse absorbante à tester dans un tube en acier de 3 cm de diamètre. Le solide a été réparti en 5 lits de 10 cm³ chacun, séparés entre eux par des tampons de laine de verre. On a passé la charge à purifier en flux ascendant dans les conditions suivantes :

Pression totale : 30 bars

Température : 50°C

Débit de charge liquide : 100 cm³/h (VVH = 2)

On a laissé passer la coupe $C_3$ pendant 10 heures. Au bout de ce temps, on a déterminé la composition du produit sortant du réacteur. Les résultats obtenus sur chacune des masses sont rassemblés dans le tableau 2.

Tableau 2.

| Hydrocarbures | | Masse n° 1 | Masse n° 2 | Masse n° 3 | Masse n° 4 |
|---|---|---|---|---|---|
| Propane | % pds | 12 | 12 | 12 | 12 |
| Propylène | " | 83 | 83,2 | 83,2 | 83,2 |
| Propyne | " | 1,6 | 2,5 | 2,5 | 2,5 |
| Propadiène | " | 1,8 | 2,1 | 2,1 | 2,1 |
| $C_4$ | " | 0,3 | 0,2 | 0,2 | 0,2 |
| $C_5^+$ | " | 1,3 | < 0,1 | < 0,1 | < 0,1 |
| COS ppm | | < 0,1 | < 0,1 | < 0,1 | < 0,1 |
| As ppb | | < 10 | < 10 | < 10 | < 10 |

On voit, dans ce tableau, que la masse n°1 préparée sur un support d'une acidité supérieure à 40 J/g produit une quantité appréciable de polymères ($C_5^+$). Ceci est un gros inconvénient car cela, non seulement, nuit au rendement de l'opération mais, encore, provoque un bouchage progressif des pores de la masse absorbante diminuant, ainsi, son efficacité d'absorption.

Afin de comparer l'efficacité d'adsorption des différents solides, les tests ont été prolongés pendant plus de 500 heures.

Au bout de 500 heures, on a mesuré la teneur en arsenic et en COS du produit sortant du réacteur. Les concentrations obtenues sont résumées dans le tableau 3.

5

Tableau 3.

| Masse | As (ppm) | COS (ppm) |
|-------|----------|-----------|
| 1 | 0,3 | 0,5 |
| 2 | < 0,01 | < 0,1 |
| 3 | < 0,01 | < 0,1 |
| 4 | < 0,01 | < 0,1 |

On voit que la masse n°1 n'est plus totalement efficace au bout de ce temps, puisqu'elle laisse passer une quantité notable d'arsenic et de COS, contrairement à ce qui est observé avec les masses utilisées dans l'invention.

De plus, on constate que la masse n°1 continue, au bout de 500 heures, à produire des hydrocarbures en $C_5^+$ (de l'ordre de 1,3%), ce qui nuit au rendement en coupe $C_3$, contrairement à ce qui est observé sur les masses 2, 3 et 4 de l'invention.

Au bout de 517 heures, le test a été arrêté et, après séchage de la masse par balayage à l'azote, on a déchargé le solide lit par lit. Sur chacun des lits, on a mesuré la teneur pondérale en arsenic et en soufre. Les résultats obtenus sur chacune des masses sont rassemblés dans le tableau 4.

Tableau 4.

| | Masse n° 1 | | Masse n° 2 | | Masse n° 3 | | Masse n° 4 | |
|------|------|------|------|------|------|------|------|------|
| Lits | % poids | | % poids | | % poids | | % poids | |
| | As | S | As | S | As | S | As | S |
| 1er | 0,30 | 0,27 | 0,66 | 0,70 | 0,71 | 0,75 | 0,68 | 0,73 |
| 2ème | 0,23 | 0,21 | 0,30 | 0,22 | 0,27 | 0,17 | 0,29 | 0,19 |
| 3ème | 0,18 | 0,16 | 0,04 | <0,05 | 0,02 | <0,05 | 0,03 | <0,05 |
| 4ème | 0,11 | 0,10 | <0,02 | <0,05 | <0,02 | <0,05 | <0,02 | <0,05 |
| 5ème | 0,08 | 0,07 | <0,02 | <0,05 | <0,02 | <0,05 | <0,02 | <0,05 |

On constate que, sur les masses préparées selon l'invention, la quasi totalité de l'arsenic et du soufre a été fixée sur les deux premiers lits. Les trois cinquièmes de ces masses restent donc encore disponibles pour fixer ces impuretés au bout de 517 heures. On peut donc s'attendre à des durées de fonctionnement efficaces très importantes. En revanche, sur la masse n°1, préparée avec un support non approprié, l'ensemble du solide est contaminé par l'arsenic et le soufre, ce qui est l'indication que, à la fin du test, l'adsorption de ces impuretés n'est déjà plus complète, comme l'ont montré les résultats du tableau 1.

Exemple 2

Dans cet exemple, on a préparé une nouvelle masse de captation sur le même support que celui de la masse n°3 et contenant également 16% de PbO, mais le sel de plomb utilisé a été le nitrate au lieu de l'acétate. On a chargé le réacteur comme dans l'exemple 1 et on a traité la même charge dans les mêmes conditions que celles de l'exemple 1. La durée de l'essai fut également de 517 heures. Au bout de ce temps, le solide a été déchargé et analysé lit par lit. Les concentrations en arsenic et en soufre, trouvées sur chacun des lits, sont rapportées dans le tableau 5.

### Tableau 5.

| Masse absorbante | Arsenic % poids | Soufre % poids |
|---|---|---|
| 1er lit | 0,52 | 0,54 |
| 2ème lit | 0,25 | 0,21 |
| 3ème lit | 0,13 | 0,11 |
| 4ème lit | 0,08 | 0,06 |
| 5ème lit | 0,02 | <0,05 |

Si l'on compare ces résultats à ceux du tableau 4 (masse n°3), on voit que, au bout du même temps, les quatre cinquièmes de la masse sont contaminés, au lieu d'environ deux cinquièmes sur la masse préparée avec l'acétate de plomb.

Les résultats sont, cependant, meilleurs que ceux de la masse n°1 dans l'exemple 1.

De plus, comme dans l'exemple 1, la masse préparée selon l'invention laisse inchangée la composition de la coupe hydrocarbonée ; il n'y a, ainsi, aucune formation détectable d'hydrocarbures lourds ($C_5^+ < 0,1\%$).

### Exemple 3 (comparaison)

Dans cet exemple, on traite la même coupe que celle de l'exemple 1 dans les mêmes conditions de température et de débit sauf pour la pression qui est de 15 bars. A la température de 50°, la coupe C3 se trouve donc entièrement vaporisée dans le réacteur.

La masse absorbante placée dans le réacteur est la masse n°3.

Au bout de 500 heures de cette opération en phase vapeur, la teneur en arsenic et en COS du produit a été mesurée. Les concentrations sont respectivement de 0,1 ppm et de 0,2 ppm en poids, valeurs à comparer avec celles du tableau 3.

De plus après 517 heures, on décharge la masse absorbante comme dans l'exemple 1 et on mesure la teneur en arsenic et en soufre sur chacun des lits ; les résultats sont présentés dans le tableau suivant :

| Lits | % pds As | % pds S |
|------|----------|---------|
| 1er | 0,36 | 0,33 |
| 2ème | 0,28 | 0,26 |
| 3ème | 0,15 | 0,13 |
| 4ème | 0,07 | 0,06 |
| 5ème | 0,04 | 0,03 |

Si on compare ces résultats avec ceux du tableau 4 (masse n°3), on voit que lorsqu'on travaille en phase gazeuse, l'efficacité de la masse absorbante est beaucoup moins grande : en effet l'ensemble des lits est contaminé par l'arsenic et le soufre contrairement à ce qui se passe en phase liquide pour laquelle seuls les deux premiers lits sont contaminés.

## Revendications

1. Procédé pour l'élimination conjointe d'arsenic et d'oxysulfure de carbone d'une coupe d'hydrocarbures insaturés, dans lequel on met ladite coupe d'hydrocarbures au contact d'une masse d'absorption renfermant un support et de l'oxyde de plomb, caractérisé en ce que ladite mise en contact se fait avec la coupe d'hydrocarbures en phase liquide.

2. Procédé selon la revendication 1, caractérisé en ce que ladite masse résulte (a) du mélange d'une solution d'un composé soluble du plomb avec un support d'alumine de surface entre 10 et 300 $m^2/g$, de volume poreux entre 0,2 et 1,2 $cm^3/g$, de volume macroporeux, calculé en pores supérieurs à 100 nm, entre 0,1 et 0,5 $cm^3/g$ et d'acidité, mesurée par le chaleur d'adsorption d'ammoniac sous 40 kPa à 320°C, inférieure à 40 joules par gramme de support, suivi (b) d'un chauffage à 500-900°C en atmosphère renfermant de l'oxygène.

3. Procédé selon la revendication 2, dans lequel le support d'alumine a une surface entre 50 et 200 $m^2/g$ et un volume poreux total entre 0,5 et 1,2 $cm^3/g$.

4. Procédé selon la revendication 2 ou 3, dans lequel le composé soluble de plomb est l'acétate de plomb.

5. Procédé selon l'une des revendications 1 à 4, dans lequel la masse renferme 5 à 50% en poids de plomb, calculé comme PbO.

## Patentansprüche

1. Verfahren zur gleichzeitigen Entfernung von Arsen und Kohlenstoffoxisulfid aus einem Schnitt ungesättigter Kohlenwasserstoffe, wobei man den genannten Kohlenwasserstoff-Schnitt mit einer Absorptionsmasse, die einen Träger und Bleioxid aufweist, in Berührung bringt, dadurch gekennzeichnet, daß dieses in Berührung bringen mit dem Kohlenwasserstoff-Schnitt in flüssiger Phase stattfindet.

2. Verfahren nach Anspruch 1, dadurch gekennzeichnet, daß die genannte Masse aus (a) einem Gemisch einer Lösung einer löslichen Bleiverbindung mit einem Aluminiumoxidträger, einer Oberfläche von 10 bis 300 $m^2/g$ mit, einem Porenvolumen zwischen 0,2 und 1,2 $cm^3/g$, einem makroporösen Volumen gerechnet als Poren einer Größe oberhalb von 100 nm zwischen 0,1 und 0,5 $cm^3/g$ und einer Acidität, gemessen durch Ammoniakadsorptionswärme, bei 40 kPa bei 320°C, von unterhalb 40 Joules pro Gramm Träger, gefolgt (b) von einer Erhitzung auf 500 bis 900°C in einer sauerstoffhaltigen Atmosphäre, stammt.

3. Verfahren nach Anspruch 2, dadurch gekennzeichnet, daß der Aluminiumoxidträger eine Oberfläche zwischen 50 und 200 m²/g und ein Gesamtporenvolumen zwischen 0,5 und 1,2 cm³/g aufweist.

4. Verfahren nach Anspruch 2 oder 3, dadurch gekennzeichnet, daß die lösliche Bleiverbindung aus Bleiacetat besteht.

5. Verfahren nach mindestens einem der Ansprüche 1 bis 4, dadurch gekennzeichnet, daß die Masse 5 bis 50 Gew.-% Blei, gerechnet als PbO, enthält.

**Claims**

1. Process for eliminating jointly arsenic and carbon oxysulfide from an unsaturated hydrocarbon cut, comprising contacting said hydrocarbon cut in the liquid phase with an absorbing mass containing a support and lead oxide.

2. A process according to Claim 1, wherein said mass results from (a) admixing of a solution of a soluble lead compound with an alumina support with a surface from 10 to 300 m²/g, a pore volume from 0.2 to 1.2 cm³/g, a macropore volume, calculated for the pores greater than 100 nm, from 0.1 to 0.5 cm³/g and an acidity, measured by the ammonia adsorption heat under 40 kPa at 320°C, smaller than 40 joules per gram of support, and (b) heating from 500 to 900°C in an oxygen-containing atmosphere.

3. A process according to Claim 2, wherein the alumina support has a surface from 50 to 200 m²/g and a total pore volume from 0.5 to 1.2 cm³/g.

4. A process according to Claim 2, wherein the soluble lead compound is lead acetate.

5. A process according to Claim 1, wherein the mass comprises 5 to 50% lead by weight, calculated as PbO.